# EUROPÄISCHE PATENTSCHRIFT

(11) **EP 1 450 875 B1**
(45) Veröffentlichungstag und Bekanntmachung des Hinweises auf die Patenterteilung: **25.05.2005**
(21) Anmeldenummer: 02799730.3
(22) Anmeldetag: 28.11.2002
(51) Int. Cl.: A61L 31/02, A61L 31/12, A61L 31/14, A61B 17/86

(54) **GELENKIMPLANTAT SOWIE VERFAHREN ZUM HERSTELLEN EINES SOLCHEN**
ARTICULAR IMPLANT AND METHOD FOR THE PRODUCTION THEREOF
IMPLANT ARTICULAIRE ET SON PROCEDE DE REALISATION

(30) Priorität: 28.11.2001 DE 10157782
(43) Veröffentlichungstag der Anmeldung: 01.09.2004
(73) Patentinhaber: Schunk Kohlenstofftechnik GmbH, D-35452 Heuchelheim (DE)
(72) Erfinder: WEISS, Roland, 35625 Hüttenberg (DE); SCHNEWEIS, Stefan, 61279 Grävenwiesbach (DE); SCHEIBEL, Thorsten, 61231 Bad Nauheim (DE); HENRICH, Martin, 35582 Wetzlar (DE); EBERT, Marco, 35083 Wetter (DE)
(74) Vertreter: Stoffregen, Hans-Herbert, Dr. Dipl.-Phys.
(86) Internationale Anmeldenummer: PCT/EP2002/013428
(87) Internationale Veröffentlichungsnummer: WO 2003/045463

(56) Entgegenhaltungen:
- DE-A- 2 142 820
- DE-A- 3 204 700
- DE-A- 3 902 856
- DE-A- 19 730 296
- US-A- 5 981 827

## Beschreibung

Die Erfindung bezieht sich auf ein Gelenkimplantat in Stiftform, bestehend aus einem mit Kohlenstoff verdichteten kohlenstoffhaltigen Basismaterial. Ferner nimmt die Erfindung auf die Verwendung eines Implantats Bezug.

Bei Gelenkerkrankungen (Arthrose) ist es bekannt, dass diese Erkrankung mit einer Defektstelle im Gelenkknorpel verbunden ist. Knorpelneubildungen im Körper sind normalerweise nicht möglich.

Es ist jedoch aus der Literatur bekannt, dass bei derartigen Gelenkerkrankungen (Arthrosen) CFK-Stifte in das Gelenk eingesetzt werden, die zur Gewebeneubildung in der Gelenkkapsel führen sollen. Die Stifte sind mit PV-beschichteten C-Fasern umgeben, die die Gewebeneubildung (Knorpelbildung) begünstigen sollen.

Nachteilig bei diesen Verfahren ist es, dass die eingesetzten Werkstoffe nicht bio-inert sind und zu starker Partikelbildung neigen, die zu Gegenreaktionen im Körper fuhren. Außerdem sind entsprechende CFK-Stifte wegen ihrer geringen Stabilität im Bedarfsfalle relativ schwierig zu entfernen.

Nachteilig entsprechender Implantate ist es auch, dass ein Einwachsen von Gewebe nicht möglich oder erschwert ist. Häufig halten entsprechende CFK-Stifte auch nicht die gewünschten Belastungen auf Dauer aus.

Aus der DE 39 02 856 A1 ist eine Prothese wie Herzklappenprothese aus Pyro-Kohlenstoff bekannt. Dabei weist der entsprechende Formkörper ein spezifisches Gewicht zwischen 1,2 bis 1,3 g/cm³ auf.

Nach der DE 197 30 296 A1 werden in ein aus Kohlenstoffmaterial bestehendes Implantat Kohlenstoffionen implantiert, so dass die mit Blut in Berührung kommenden Flächen des Implantats mit Kohlenstoff angereichert werden.

Ein künstliches Prothesenmaterial auf Kohlenstoff-Basis, das eine poröse Strukturschicht mit einem Fasermaterial und pyrolytisch abgeschiedenen Kohlenstoff aufweist, ist aus der DE 32 04 700 A1 bekannt.

Zum Verdichten von einem faserverstärkten Verbundwerkstoff mit einer C-Matrix erfolgt nach der EP 0946 459 B1 eine chemische Gasphaseninfiltration, um einen höheren Porenfüllungsgrad zu erzielen. Entsprechende Verbundwerkstoffe können als Knochen- bzw. Gelenkprothesen verwendet werden.

Ein Kohlenstoffverbundkörper nach der WO 96/36473 kann mittels CVI verdichtet und als Bio-Implantat verwendet werden.

Der US 5,981,821 ist ein aus Kohlenstoff bestehendes Implantat bekannt, das eine poröse Struktur mit einem Porendurchmesser weniger als etwa 200 µm aufweist. Das Implantat besitzt eine Schichtstruktur, wobei ein aus Kohlenstoff bestehender Basiskörper mit einer dünnen Schicht aus diamantähnlichem Kohlenstoff abgedeckt ist. Das Implantat besitzt des Weiteren einen Dichtegradienten mit im Inneren höherer Dichte als im Oberflächenbereich.

Aufgabe der vorliegenden Erfindung ist es, ein Gelenkimplantat derart weiterzubilden, dass gewünschte physikalische und biologische Eigenschaften gegeben sind, wobei die Herstellung des Implantats selbst zu herstellungstechnischen Schwierigkeiten nicht führen soll. Dabei soll auch die Möglichkeit gegeben werden, dass im gewünschten Umfang Gewebe in das Implantat einwachsen kann und damit das Implantat als Biotemplat nutzbar ist.

Erfindungsgemäß wird das Problem durch ein Implantat der eingangs genannten Art im Wesentlichen dadurch gelöst, dass durch CVI mit Kohlenstoff verdichteter Kohlenstoff-Filz oder Kohlenstoff-Schaum einer Dichte ρ₁ mit in etwa 0,08 g/cm³ ≤ ρ₁ ≤ 0,11 g/cm³ das Basismaterial ist und dass das Implantat eine Enddichte ρ₃ mit in etwa 0,5 g/cm³ ≤ ρ₃ ≤ 0,7 g/cm³ und eine Porosität zwischen 30 % und 80 % aufweist.

Durch die Verwendung von Kohlenstoff-Filz oder Kohlenstoff-Schaummaterial, das in Streifen- oder Bahnenform vorliegen kann, wird ein definierte physikalische und chemische Eigenschaften aufweisendes Ausgangsmaterial benutzt, das mit Kohlenstoff z. B. durch CVI (Chemical Vapour Infiltration)-Verfahren verdichtet ist und somit eine hinreichende Eigensteifigkeit aufweist, um Abschnitte gewünschter Geometrie auszuschneiden und sodann weiterbearbeiten zu können. Durch das Verdichten mit Kohlenstoff kann der zu verarbeitende Abschnitt außerdem reproduzierbar definierte Dichte- und Porositätswerte erhalten.

Aus dem Material ausgeschnittene Abschnitte werden sodann bearbeitet wie geschliffen und auf Endmaß gebracht, um anschließend nach einem Reinigen und Entstauben erneut eine Koblenstoffverdichtung durch z. B. CVI durchzuführen.

So hergestellte Implantate weisen eine reproduzierbare Porosität auf, sind bio-inert und chemisch-inert. Insbesondere durch das Einstellen der Porosität ist die Möglichkeit gegeben, dass Gewebezellen im gewünschten Umfang einwachsen können. Eine Zweitbeschichtung der fertig bearbeiteten Implantate minimiert den Partikelrelease bei der Anwendung, bei Erhalt der Rauheitsgeometrie und gleichzeitiger Festigkeitserhöhung.

Als Basismaterial wird ein graphitierter Kohlenstoff-Filz mit einer Dichte im Bereich zwischen 0,08 und 0,11 g/cm³ benutzt. Das Basismaterial wird sodann mit Kohlenstoff in einem Umfang verdichtet, dass sich eine Dichte von 0,25 und 0,35 g/cm³ ergibt. Diese Vorverdichtung reicht aus, um die Implantate schädigungsfrei zu bearbeiten.

Nach der Bearbeitung und Reinigung erfolgt die Weiterverdichtung auf die gewünschte Enddichte derart, dass sich eine Dichte zwischen 0,5 und 0,7 g/cm³ ergibt. Die Dichte kann, je nach Anwendungsbedarf, in einem weiten Bereich modifiziert werden, um für das Einwachsen des gewünschten Gewebes optimale Voraussetzungen zu bieten.

Die Kohlenstoffnachverdichtung erfolgt vorzugsweise mittels CVI, um eine möglichst niedrige Tendenz zur Partikelbildung zu gewährleisten. Ein vergleichbarer Effekt wäre auch über Glaskohlenstoffbildung erzielbar.

Erfindungsgemäße eine Stiftgeometrie aufweisende Gelenkimplantate können sodann in einem Knochen eines Gelenks eingesetzt werden. Die entsprechenden Stifte weisen eine hinreichende Stabilität auf. Durch die Porosität kann das Implantat mit Faserknorpel überzogen werden, so dass das Gelenk seine ursprüngliche Funktion ausüben kann.

Eine Verwendung des Implantats ist jedoch nicht nur als Biotemplat für das Gewebe, sondern auch für das Einwachsen von Knochen in einem Gelenk geeignet. Diese Funktionen können durch das Aufbringen von Zellwachstumsfaktoren noch verbessert werden. Eine weitere Verwendung ist auch als Medikamententräger möglich, der in einem Körper implantiert werden kann, um im gewünschten Umfang medizinische Mittel abzugeben. Da das Implantat bio-inert und chemisch-inert ist, ist weder ein Abstoßen möglich noch kann das Medikament verändert werden.

Zum Herstellen eines erfindungsgemäßen Gelenkimplantats sind folgende Verfahrensschritte vorgesehen:
- Vorverdichten eines kohlenstoffhaltigen porösen Basismaterials mit Kohlenstoff,
- Abtrennen zumindest eines Abschnitts aus dem Basismaterial,
- Bearbeiten des Abschnitts zur Erzielung einer gewünschten Implantatgeometrie und
- Nachverdichten des bearbeiteten und gereinigten Abschnitts mit Kohlenstoff.

Insbesondere ist vorgesehen, dass das Abtrennen des Abschnitts aus dem Basismaterial durch Sägen erfolgt. Das anschließende Bearbeiten sollte ein spitzenloses Schleifen sein. Die Reinigung des abgesägten, geschliffenen und sodann abgelängten Abschnitts sollte insbesondere mittels Ultraschall erfolgen.

Unabhängig hiervon wird als Basismaterial Insbesondere ein Kohlenstoff-Filz wie graphitierter Kohlenstoff-Filz verwendet.

Auch ein Kohlenstoffschaum kann als Basismaterial, das ebenfalls verdichtet wird, verwendet werden. Dabei ist es erforderlich, dass der Kohlenstoffschaum eine hinreichende Steifigkeit aufweist.

Andere Kohlenstoffmaterialien sind ebenfalls dann einsetzbar, wenn die Einstellung einer definierten und hohen Porosität möglich ist.

Insbesondere wird ein graphitierter Kohlenstoff-Filz als Basismaterial verwendet, der bei einer Dicke d mit 4mm ≤ d ≤ 7mm ein Flächengewicht zwischen 450 und 650 g/m² aufweist. Die Zugfestigkeit eines entsprechenden Filzes sollte in Längsrichtung in etwa 0,09 bis 0,11 N/mm² und in Querrichtung 0,05 bis 0,07 N/mm² aufweisen. Die spezifische Dichte des Filzmaterials sollte des Weiteren im Bereich zwischen 0,08 und 0,11 g/cm³ liegen.

Das Basismaterial wird sodann vorzugsweise durch CVI (Chemical Vapour Infiltration)-Verfahren verdichtet, um eine Dichte im Bereich zwischen 0,25 und 0,35 g/cm³ zu erzielen.

Aus einem entsprechend verdichteten Basismaterial werden sodann Abschnitte ausgeschnitten, die jeweils geringfügig größer als das Implantat in seiner Endgeometrie sind.

Sodann erfolgen übliche Bearbeitungsvorgänge wie Schleifen, um den Abschnitt auf das gewünschte Endmaß zu bearbeiten.

Nach dem Reinigen und Entstauben erfolgt eine weitere Kohlenstoffverdichtung durch z.B. einen CVI-Prozess, um eine Enddichte von in etwa 0,35 bis 0,70 g/cm³ zu erreichen. Ein so hergestelltes Implantat weist eine gewünschte Porosität auf und ist bio-inert und chemisch-inert.

Insbesondere durch die gezielte Nachverdichtung ist eine Porosität erreichbar, um ein Einwachsen von Gewebezellen zu erreichen. Bevorzugterweise sollte das Implantat eine Porosität zwischen 30 % und 80 %, insbesondere im Bereich zwischen 50 % und 70 % aufweisen. Durch die Kohlenstoffverdichtung nach der Endbearbeitung erhält das Implantat eine hervorragende Abriebfestigkeit, so dass ein gefahrlöser Einsatz sichergestellt ist. Das Implantat ist zudem hochrein.

Entsprechende Implantate, sofern diese eine stiftförmige Geometrie aufweisen und in Gelenken eingesetzt werden sollen, können eine Länge zwischen 10 und 15 mm und einen Durchmesser zwischen 1 und 5 mm aufweisen.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden bevorzugten Ausführungsbeispielen.

Es zeigen:
- Fig. 1: eine Prinzipdarstellung eines Gelenkes,

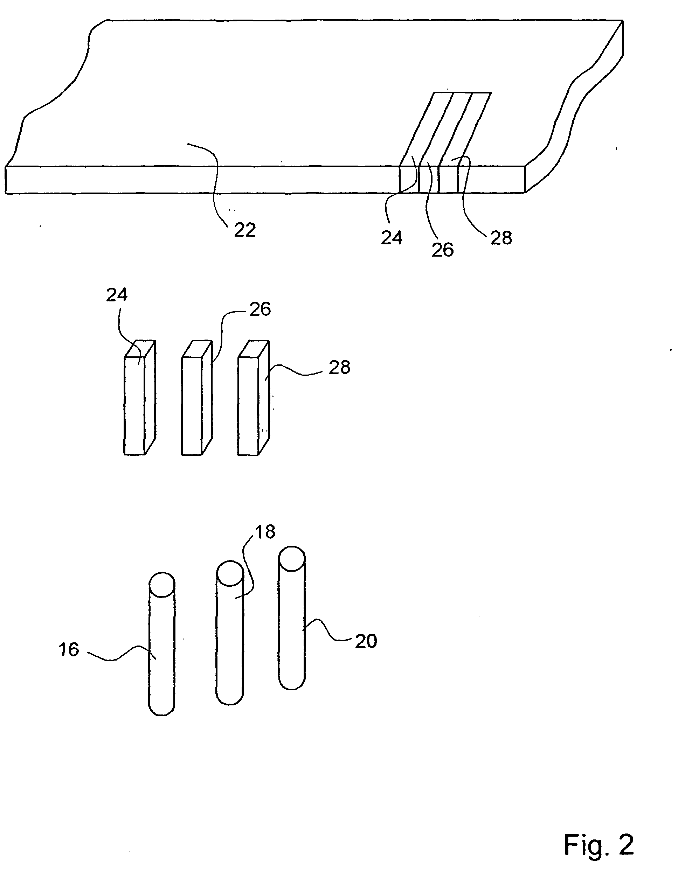

- Fig. 2: eine Prinzipdarstellung eines Arbeitsablaufes zur Herstellung von Implantaten und
- Fig. 3: ein Flussdiagramm.

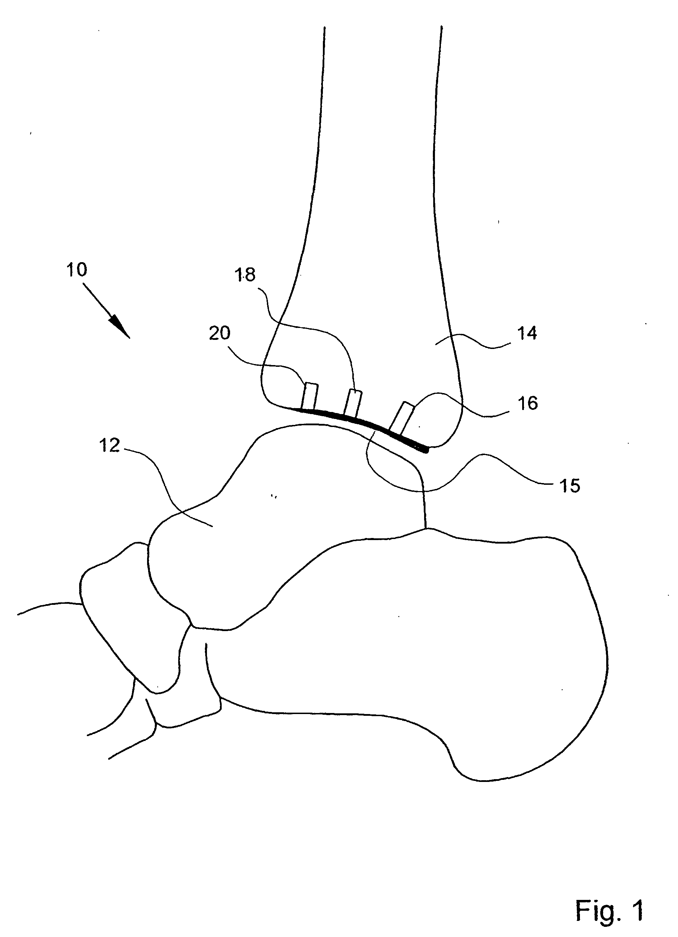

In Fig. 1 ist rein prinzipiell ein Gelenk 10 mit Knochen 12, 14 dargestellt, die zueinander verschiebbar sind. Im Falle des Auftretens von Gelenkerkrankungen, Knorpelschädigungen, die vorwiegend bei einem Mißverhältnis zwischen Beanspruchung und Beschaffung bzw. Leistungsfähigkeit der einzelnen Gelenkanteile und Gelenkgewebe entstehen, ist es erforderlich, dass in das Gelenk 10 Implantate 16, 18, 20 eingebracht werden, die zur Neubildung von Gelenkknorpel führen, um eine Entlastung der aufeinandergleitenden Knochen 12, 14 zu erzielen. Hierzu werden die Implantate 16, 18, 20 in Form von Stiften in einen der Knochen - im Ausführungsbeispiel in den Knochen 14 - eingesetzt, wobei deren Enden im Knochen 14 eingesenkt werden, so dass sich auf Gelenkfläche 15 faserhaltiger Gelenkknorpel neu bilden kann, der die Gelenkfunktionsfähigkeit erheblich verbessert. Gleichzeitig soll dabei die Möglichkeit bestehen, dass in die Implantate 16, 18, 20 Gewebe einwächst.

Entsprechende stiftförmige Implantate 16, 18, 20 werden nach der erfindungsgemäßen Lehre aus einem kohlenstoffhaltigen Basismaterial wie insbesondere graphitiertem Kohlenstoff-Filz 22 hergestellt, das bahnenförmig vorliegen kann und aus dem an die Endgeometrie der Implantate 16, 18, 20 ausgerichtete Abschnitte 24, 26, 28 ausgeschnitten werden. Damit das Filzmaterial 22 eine hinreichende Steifigkeit erhält, um ein Bearbeiten zu ermöglichen, wird dieses zuvor mit Kohlenstoff verdichtet. Hierdurch ergibt sich ein Hartfilz, aus dem die Abschnitte 24, 26, 28 problemlos herausgeschnitten werden können.

Der Filz 22 weist im ursprünglichen Zustand vorzugsweise eine Dichte zwischen in etwa 0,08 bis 0,11 g/cm³ auf. Nach dem Verdichten mit Kohlenstoff, insbesondere durch CVI-Verfahren, weist der Hartfilz bevorzugterweise eine Dichte von in etwa 0,3 g/cm³ auf.

Die aus dem Hartfilz herausgeschnittenen Abschnitte 24, 26, 28 werden sodann geschliffen und auf Endmaß bearbeitet, so dass sich die Endform der Implantate 16, 18, 20 ergibt. Die entsprechenden auf Endmaß bearbeiteten Abschnitte 24, 26, 28 werden gereinigt und entstaubt, um erneut mit Kohlenstoff verdichtet zu werden, wobei ebenfalls bevorzugterweise das CVI-Verfahren zum Einsatz gelangt. Die entsprechenden Stifte 16, 18, 20 sind im erforderlichen Umfang porös, bio-inert und chemisch-inert. Durch die Porosität, die vorzugsweise im Bereich zwischen 50 % und 70 % liegen sollte, besteht die Möglichkeit dass Gewebezellen einwachsen können.

Typische Abmessungen von entsprechenden stiftförmigen Implantaten 16, 18, 20 sind Längen zwischen 10 und 15 mm und Durchmesser von 1 bis 5 mm.

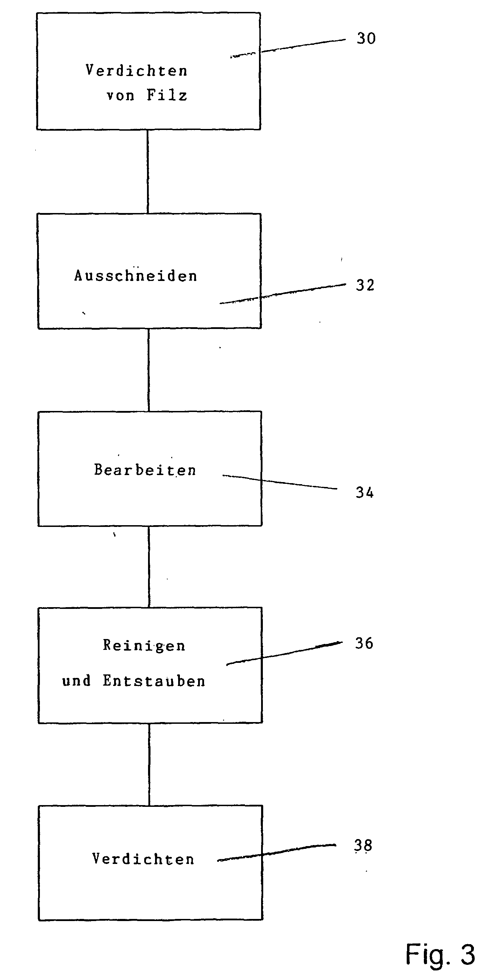

Die Verfahrensschritte selbst werden noch einmal an Hand des Flussdiagramms gemäß Fig. 3 verdeutlicht. So wird zunächst vorzugsweise von einer Rolle der Filz 22 abgezogen, wobei sich die Dicke des Filzes zwischen 4 und 10 mm belaufen sollte. Der Filz wird sodann mittels Kohlenstoff vorzugsweise im CVI-Verfahren verdichtet (Verfahrensschritt 30). Dabei soll eine Verdichtung in einem Umfang erfolgen, dass der verdichtete Filz, der ein Hartfilz bildet, im Vergleich zum Ausgangsfilz eine vorzugsweise 2- bis 4-fach höhere Dichte aufweist.

Aus dem so gewonnenen Hartfilz werden in einem nachfolgenden Verfahrensschritt 32 die Abschnitte 24, 26, 28 ausgeschnitten, um diese sodann in einem Verfahrensschritt 34 zu bearbeiten, um die gewünschte Endgeometrie zu erzielen. Nach dem Bearbeiten erfolgt in einem Verfahrensschritt 36 ein Reinigen und Entstauben der endbearbeiteten Abschnitte, um diese sodann in einem weiteren Verfahrensschritt 38 erneut mit Kohlenstoff, vorzugsweise durch CVI-Verfahren zu verdichten. Dabei sollte eine Gesamtverdichtung derart erfolgen, dass sich eine Porosität zwischen 50 und 70 % ergibt, wodurch die Möglichkeit geschaffen wird, dass in die Implantate 16, 18, 20 Gewebezellen einwachsen.

Unabhängig hiervon weisen die entsprechenden Implantate 16, 18, 20 eine hohe mechanische Stabilität auf. Auch sind Implantate 16, 18, 20 bio-inert und chemisch-inert.

Ist die erfindungsgemäße Lehre an Hand von insbesondere für Gelenke bestimmten Implantaten erläutert worden, so soll hierdurch eine Einschränkung der erfindungsgemäßen Lehre nicht erfolgen. Vielmehr können die entsprechenden Implantate auch z. B. als Medikamenten - und Zellwachstumsfaktoren eingesetzt werden.

Des Weiteren ist darauf hinzuweisen, dass es nicht zwingend erforderlich ist, dass das Ausgangsmaterial ein graphitierter Kohlenstoff-Filz ist. Vielmehr kann z.B. ein Kohlenstoffschaum verwendet werden, sofern dieser eine hinreichende Eigensteifigkeit aufweist.

## Patentansprüche

1. Gelenkimplantat (16, 18, 20) in Stiftform, bestehend aus einem mit Kohlenstoff verdichteten kohlenstoffhaltigen Basismaterial (22),
**dadurch gekennzeichnet,**
**dass** durch CVI mit Kohlenstoff verdichteter Kohlenstoff-Filz oder Kohlenstoff-Schaum einer Dichte ρ₁ mit in etwa 0,08 g/cm³ ≤ ρ₁ ≤ 0,11 g/cm³ das Basismaterial (22) ist und
**dass** das Implantat (16, 18, 20) eine Enddichte ρ₃ mit in etwa 0,5 g/cm³ ≤ ρ₃ ≤ 0,7 g/cm³ und eine Porosität zwischen 30 % und 80 % aufweist.

2. Gelenkimplantat nach Anspruch 1,
**dadurch gekennzeichnet,**
**dass** das Implantat (16, 18, 20) eine Porosität zwischen 50 % und 70 % aufweist.

3. Verwendung des Implantats nach zumindest Anspruch 1 in der Herstellung eines Medikamententrägers.

4. Verwendung des Implantats nach zumindest Anspruch 1 in der Herstellung eines Biotemplats.

## Claims

1. Articular implant (16, 18, 20) in pin form, consisting of a carbon-containing base material (22) densified with carbon, **characterised in that** carbon felt or carbon foam densified with carbon by means of CVI and having a density ρ₁, wherein substantially 0.08 g/cm³ ≤ ρ₁ ≤ 0.11 g/cm³, is the base material (22) and **in that** the implant (16, 18, 20) exhibits a final density ρ₃, wherein substantially 0.05 g/cm³ ≤ ρ₃ ≤ 0.7 g/cm³, and a porosity between 30% and 80%.

2. Articular implant according to claim 1, **characterised in that** the implant (16, 18, 20) exhibits a porosity between 50% and 70%.

3. Use of the implant according to at least claim 1 in the production of a medicine carrier.

4. Use of the implant according to at least claim 1 in the production of a biotemplate.

## Revendications

1. Implant articulaire (16, 18, 20) sous forme de pivot, constituée d'un matériau de base contenant du carbone et densifié avec du carbone (22),
**caractérisé en ce que**
le matériau de base (22) est du feutre de carbone ou de la mousse de carbone dénsifié(e) par infiltration chimique en phase vapeur d'une densité ρ₁ d'environ 0,08 g/cm³ ≤ ρ₁ ≤ 0,11 g/cm³, et l'implant (16, 18, 20) présente une densité finale ρ₃ d'environ 0,5 g/cm³ ≤ ρ₃ ≤ 0,7 g/cm³ et une porosité comprise entre 30 % et 80 %.

2. Implant articulaire selon la revendication 1,
**caractérisé en ce que**
l'implant (16, 18, 20) présente une porosité comprise entre 50 % et 70 %.

3. Utilisation de l'implant selon au moins la revendication 1, dans la fabrication d'un support pour médicament.

4. Utilisation de l'implant selon au moins la revendication 1, dans la fabrication d'un biogabarit.
